# EUROPEAN PATENT SPECIFICATION

(11) **EP 0 515 550 B1**
(45) Date of publication and mention of the grant of the patent: **17.12.1997**
(21) Application number: 91905244.9
(22) Date of filing: 14.02.1991
(51) Int. Cl.: A61K 38/00, A61K 31/725

(54) **HEPARIN NEUTRALIZATION WITH PLATELET FACTOR 4**
HEPARINNEUTRALISIERUNG DURCH BLUTPLÄTTCHENFAKTOR
NEUTRALISATION DE L'HEPARINE AU MOYEN DU FACTEUR DE PLAQUETTES 4

(30) Priority: 16.02.1990 US 480873
(43) Date of publication of application: 02.12.1992
(73) Proprietor: REPLIGEN CORPORATION, Cambridge, MA 02139 (US)
(72) Inventor: COOK, Jacquelynn, J., Lansdale, PA 10446 (US); NIEWAROWSKI, Stefan, Narbeth, PA 19072 (US); MAIONE, Theodore E., Wakefield, MA 01180 (US)
(74) Representative: Deans, Michael John Percy
(86) International application number: US9101051
(87) International publication number: WO9112017

(56) References cited:
- US-A- 4 844 895
- BRITISH HEART JOURNAL, Vol. 52, No. 1, issued July 1984; W.S. WALKER et al.: "Successful Cardiopulmonary Bypass in Diabetics with Anaphyloctoid Reactions to Protamine", pages 112-114, see entire document
- THROMBOSIS RESEARCH, Vol. 46, No. 1, issued 1987; J.N. SHANBERGE et al.: "Interrelationship of Protamine and Platelet Factor e in the Neutralization of Heparin", pp. 89-100
- BRITISH JOURNAL OF HAEMATOLOGY, Vol. 38, issued 1978; R. MICHALSKI et al.: "Neutralization of Heparin in Plasma by Platelet Factor 4 and Protamine Sulphate", pages 561-571, see entire document
- BIOCHEMICAL JOURNAL, Vol. 218, issued 1984; D.A. LANE et al.: "Anticoagulant Activities of Heparin Oligosaccharides and their Neutralization by Platelet Factor 4", pages 725-732, see entire document
- SEMINARS IN HEMATOLOGY, Vol. 22, No. 2, issued April 1985; J.C. HOLT et al.: "Biochemistry of alpha Granule Proteins", pages 151-163, see entire document
- THE JOURNAL OF BIOLOGICAL CHEMISTRY, Vol. 251, No. 14 issued 25 July 1976; "Purification and Binding Properties of Human Platelet Factor Four", pages 4273-4282, see entire document
- EUROPEAN JOURNAL OF CLINICAL INVESTIGATION, Vol. 17, No. 1, issued February 1987, G. CELLA et al, "Human Platelet Factor 4 and Protamine Sulphate Interaction with Glycosaminoglycans in the Rabbit", pp. 548-554
- THROMBOSIS RESEARCH, Vol. 12, No. 5, issued April 1978; J. DAWES et al.: "The Release, Distribution, and Clearance of Human beta-Thromboglubulin and Platelet Factor 4", pages 851-861
- PROCEEDINGS OF THE NATIONAL ACADEMY OF SCIENCES (USA), Vol. 78, No. 7, issued July 1981; T.F. DEUEL et al.: "Platelet Factor 4 is Chemotactic for Neutrophils and Monocytes", pages 4584-4587
- TRANSCRIPTS OF THE AMERICAN SOCIETY OF ARTIFICAL INTERNAL ORGANS, Vol. 28, issued 1982; W. FLICKER et al.: "Platelet Factor Release Following Heparin Administration and During Extracorporeal circulation", pages 431-436
- THE JOURNAL OF BIOLOGICAL CHEMISTRY, Vol. 252, No. 18, issued 25 September 1977; M. HERMODSON et al.: "Isolation, Crystallization, and Primary Amino Acid Sequence of Human Platelet Factor 4", pages 6276-6279
- THE JOURNAL OF BIOLOGICAL CHEMISTRY, Vol. 251, No. 2, issued 25 January 1976; S.P. LEVINE et al.: "Human Platelet Factor 4: Purification and Characterization by Affinity Chromatography", pp. 324-328
- BLOOD, Vol. 61, No. 6, issued June 1983, A.K. RAO et al, "Effect of Heparin on the In Vivo Release and Clearance of Human Platelet Factor 4", pages 1208-1214
- AMERICAN JOURNAL OF PHYSIOLOGY, Vol. 251, No 4 part 1, issued 1986, B. RUCINSKI et al.: "Clearance of Human Platelet Factor 4 by Liver and Kidney: its Alteration by Heparin", pages H800-H807
- SEMINARS IN THROMBOSIS AND HEMOSTASIS, Vol. 11, No. 1, issued 1985; D.A. WALZ et al.: "In Vivo Studies on the Binding of Heparin and Its Fractions with Platelet Factor 4", pages 40-47
- BIOCHEMICAL JOURNAL, Vol. 234, issued 01 March 1986; S.W. COWAN et al.: "Binding of Heparin to Human Platelet Factor 4", pages 485-488
- THE JOURNAL OF BIOLOGICAL CHEMISTRY, Vol. 261, No. 9, issued 25 March 1986; D. A. LANE et al.: "Neutralization of Heparin-related Saccharides by Histidincerich Glycoprotein and Platelet Factor 4", pages 3980-3986
- Science, Vol. 247, issued January 1990; T.E. MAIONE et al.: "Inhibition of Angiogenesis by Recombinant Human Platelet Factor 4 and Related Peptides", pp. 277-279

## Description

The invention relates to the use of Platelet factor-4 (PF4) or fragments thereof in the manufacture of medicaments for neutralization of heparin.

Applications of advancing medical technology, such as cardiopulmonary bypass, are associated with a variety of complications. Even in short term use, blood, oxygenators produce sufficient activation of clotting pathways to require the use of heparin to inhibit blood coagulation.

An invariable complication of such surgical procedures is a hemorrhagic state manifested by a prolonged bleeding time, causes of which include failure to adequately neutralize heparin and the continuous stimulation of platelets, as manifested by a fall in platelet count, stimulation of thromboxane synthesis and release of platelet granule constituents. (See Colman, J. Anesthesiology, vol. 66 (5), May, 1987, p. 595). Reversal of heparin is required to restore normal coagulation status and reduce post-operative blood loss.

Protamine is an arginine-rich polypeptide (32 amino acids from salmon) commonly used at the conclusion of cardiovascular surgical procedures to neutralize the anticlotting effects of heparin. The use of protamine, however, has been linked to several post-surgical complications, some of which are postoperative systemic hypotension, allergic reactions, catastrophic pulmonary vasoconstriction, acute pulmonary hypertension, complement activation, noncardiogenic pulmonary edema, decreased cardiac output (later event), and thrombocytopenia/leukopenia.

The underlying biochemical basis for these physical complications is poorly understood, but allergic reactions to protamine, have been well documented. Since protamine, usually isolated from fish, can be recognized as a foreign protein by the human immune system, patients with prior protamine exposure are at particular risk during subsequent exposures (Just Viera, J.O., Amer. Surgeon 50, (**1984**), 151-163). Additionally, studies suggest that a non-immunological pathway via complement activation may be responsible for many of the acute reactions observed during protamine reversal of heparin anticoagulation.

To avoid the use of protamine a number of approaches have been proposed. Construction of bypass circuits with materials that do not activate the coagulation cascade have been suggested, as well as the use of non-heparin anticoagulate preparations. Neutralizing agents for heparin other than protamine are also currently being sought. Horrow, "Effective Hemostasis in Cardiac Surgery", chap. 2, Ellison and Jobes, Eds, in press, 1988. All of these alternatives, presently in various stages of research, have yet to reveal a suitable substitute for protamine that has gained widespread acceptance.

PF4 is a well characterized heparin-binding protein found in human plasma at concentrations of 5-20ng/ml. In plasma, in in vitro studies, PF4 has been demonstrated to reverse the effect of heparin on clot formation. Michalski, Brit. J. Haematol, 38, 561-571 (1978).

In vivo experiments demonstrate that PF4 is cleared rapidly from circulation in both rats and rabbits. This rapid clearance is due to PF4 binding to endothelial cells. PF4 bound by endothelial cell surfaces can be released into the bloodstream by subsequent administration of heparin. In humans, a similar rise in circulating PF4 concentration is observed following heparin administration.

Platelet concentrate has been shown to have heparin neutralizing activity when administered to humans after bypass surgery, and the effect was attributed to PF4. Walker, Br. Heart J. 52:12-14 (1984).

Further relevant background art documents are: Br. Heart J. 52, 112-114 (1984); Thrombosis Research 46, 89-100 (1987); Biochem. J. 218, 725-732 (1984); Seminars in Hematology 22, 151-163 (1985); Eur. J. Clin. Invest. 17, 548-554 (1987); Trans. Am. Soc. Artif. Intern. Organs 28, 431-436 (1982); Blood 61, 1208-1214 (1983); J. Biol. Chem. 252, 6276-6297 (1977).

In a first aspect of the invention, we provide use of purified PF4 or a heparin binding fragment thereof for the manufacture of a medicament for treatment of an undesirably high level of circulating heparin in a mammal caused by administration of heparin in connection with a medical procedure.

Heparin binding fragments are those containing the heparin binding region, amino acids 61 to 66.

The term "purified" as used herein refers to PF4 or recombinant PF4 (rPF4) which is 95% or greater, by weight, PF4 or rPF4, i.e., is substantially free of other proteins, lipids, and carbohydrates with which it is naturally associated.

A purified protein preparation will generally yield a single major band on a polyacrylamide gel for each subunit of PF4 or rPF4. Most preferably, the purified PF4 or rPF4 used in the compositions of the invention is pure as judged by amino-terminal amino acid sequence analysis.

We have discovered that normal coagulation status in mammals treated with heparin may be restored after administration of purified PF4 without substantial depletion of platelets or leukocytes or other deleterious effects often associated with the commonly used heparin neutralizing agent, protamine.

In various aspects of the invention: The PF4 or fragment is rPF4 or a fragment thereof. The PF4 or fragment is synthetic PF4 or a fragment thereof. The heparin binding fragment of PF4 contains amino acids 61 to 66. The fragment is C-41. In use, PF4 or a fragment thereof is administered in an amount sufficient to neutralize heparin administered at more than 50 units/kg of body weight. This amount is about 0.1 to 20 mg/kg of body weight, preferably 6 mg/kg of body weight.

In a second alternative aspect of the invention, we provide use of purified PF4 or a heparin binding fragment thereof for the manufacture of a medicament for restoring a normal coagulation status in a patient after administration of heparin, without substantial depletion of platelets or leukocytes.

In a third and further alternative aspect, we provide use of purified PF4 or a heparin binding fragment thereof for the manufacture of a medicament for neutralization of heparin administered to a patient to inhibit coagulation during a medical treatment.

In a yet further alternative aspect of the invention, we provide the combination of heparin and purified PF4 or a heparin binding fragment thereof for the spaced sequential use in the treatment of a patient during a medical procedure, said heparin inhibiting coagulation and said purified PF4 or heparin binding fragment thereof neutralizing said heparin.

In various aspects of the invention: The medical procedure involves heart surgery. The medical procedure requires a cardiopulmonary bypass. The medical procedure is an organ transplantation. The medical procedure is a kidney dialysis. The medical procedure is a catheterization. The catheterization is cardiac catheterization. The catheterization is for angioplasty. The medical procedure is for prevention of blood clots formation.

We first briefly describe the drawings.

Fig. 1 is a graph showing platelet count, white blood cell count, and activated partial thromboplastin time (APTT) in a rat treated with heparin, as a function of time after injection of protamine sulphate.

Fig. 2 is a graph showing platelet count, white blood cell count, and APTT in a rat treated with heparin as a function of time after injection of rPF4.

Figs. 3 to 5 are bar graphs illustrating variations in white blood cell count, platelet count, and APTT of rats treated with heparin, after administration of a control solution (Fig. 3), protamine sulfate (Fig. 4), and rPF4 (Fig. 5).

### PF4

As is described in commonly owned U.S. patent application Serial No. 451,021, filed December 27, 1989, now U.S. Patent 5,086,164, the entire contents of both applications being hereby incorporated by reference, human PF4 can be produced by recombinant methods in E. coli. A synthetic gene was constructed based on the known PF4 amino acid sequence, and a recombinant fusion protein was expressed which could be purified by heparin-agarose affinity chromatography after chemical cleavage of non-PF4 amino acids from the recombinant fusion protein.

The proteins are cleaved and extracted, and the extracts purified using Heparin-agarose chromatography, removing contaminating proteins with 0.6 M NaCl and diluting with 1.2 M NaCl. Eluted PF4 is dialyzed into 20 mM acetate buffer, pH 4.0, and then further purified by reverse phase HPLC.

As discussed in the above applications, heparin binding can be attributed to the region of the peptide containing residues 61 to 66.

### In Vitro Experiments

In vitro experiments were conducted to compare the effects of protamine and PF4 for a preliminary assessment of whether PF4 might be a suitable substitute. Additionally human platelet-derived PF4 and rPF4 were compared.

In all properties examined, human PF4 and rPF4 showed essentially identical characteristics. PF4 isolated from platelets and recombinant E. coli cells were compared for their abilities to prevent heparin inhibition of Factor Xa (essential for the production of thrombin) in vitro. The two protein preparations showed identical capacity for neutralization of heparin, with 1 nmol of protein neutralizing approximately 0.25nmol (0.1 unit) heparin in this assay.

The abilities of protamine and rPF4 to prevent heparin-inhibition of the coagulation enzyme Factor Xa were compared. The details of the assay are described by Denton et al, 1983, Biochem. J. 209:455. In the Factor Xa assay, 0.5 units of heparin inhibited enzymatic activity almost 90%. At low concentrations, both rPF4 and protamine partially restored Factor Xa activity, but at concentrations greater than 1 nM, rPF4 was clearly more effective in preventing Factor Xa inhibition.

In experiments carried out under conditions similar to the Factor Xa experiments, thrombin was inhibited 99% by 0.25 units of heparin. Both rPF4 and protamine completely prevented heparin inhibition at 1 nM and showed no significant difference in their molar dose responses.

Neutralization of heparin in human plasma was also examined. The activated partial thromboplastin time (APTT) of normal human plasma was increased from its base level of approximately 30 seconds to 195 seconds by the addition of 1 unit/ml of heparin. When added during the incubation preceding the addition of the activated thromboplastin, rPF4 and protamine were equally effective in restoring the APTT to its heparin-free level.

Similar studies and results may be obtained using certain fragments of PF4 or rPF4 that include the heparin binding region, residues 61-66. For example, C-41, a 41 amino acid C-terminal peptide of PF4 described in U.S. Patent Application Serial No. 295,555, incorporated above, demonstrated the ability to neutralize heparin in vitro using the Factor Xa assay.

### In Vivo Experiments

In Vivo experiments were performed on rats to compare the heparin neutralizing activity of protamine and PF4 and to investigate the effect of PF4 on platelet and white blood cell count.

Sprague Dawley rats (225g) were anesthetized with pentobarbital, the trachea was intubated, and the right femoral vein and carotid artery were cannulated. The intravenous infusion of heparin was followed by infusion of either protamine or rPF4. Arterial blood samples were taken prior to and immediately after heparin infusion and at several time points following the infusion of the neutralizing agent. Controls were conducted with Ringer's solution in place of heparin and the experimental agents. Samples were used for the following determinations: clotting time, platelet count, and white blood cell count.

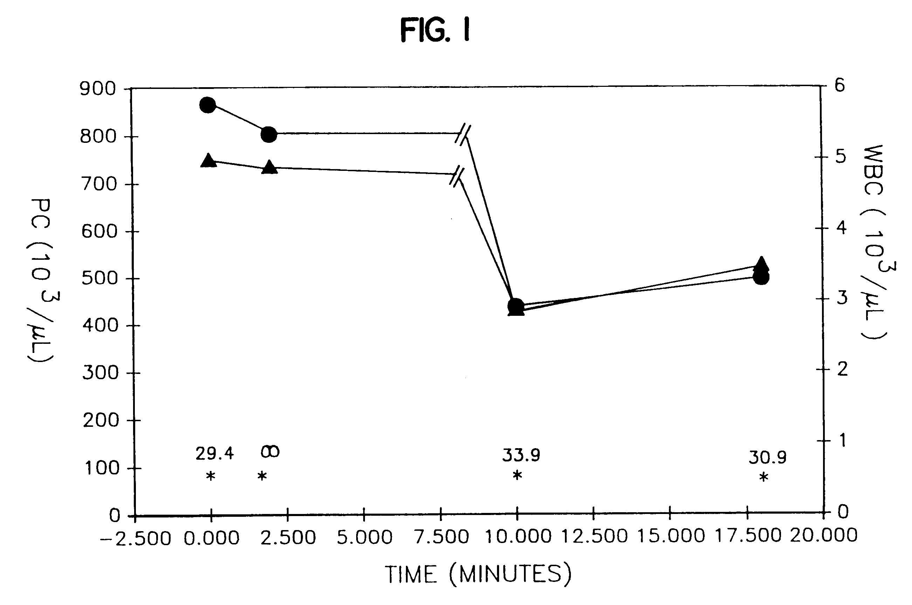

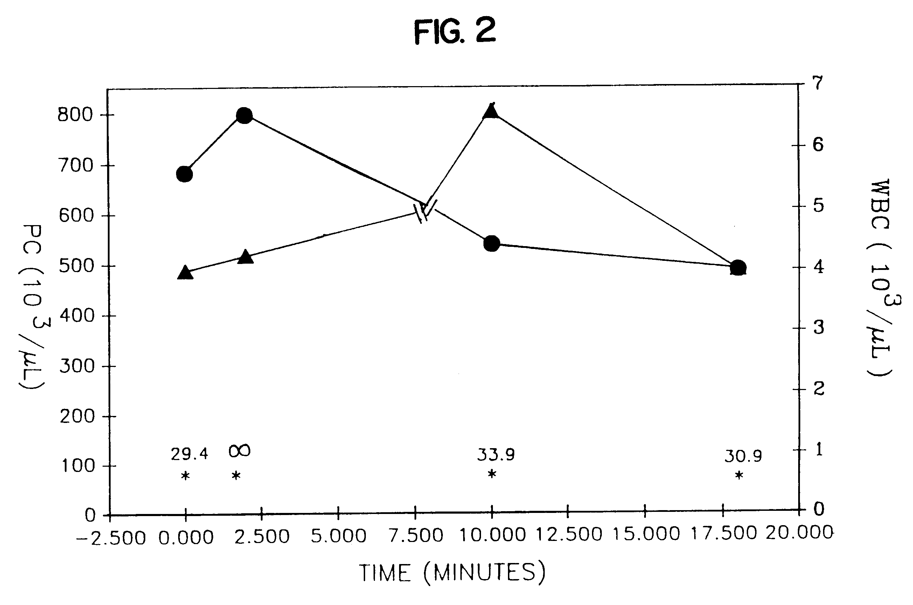

Referring to Figs. 1 and 2, data are shown from a typical experiment comparing the platelet count, white blood cell count and the APTT in rats when injected with heparin followed by protamine sulfate (Fig. 1) and when injected with heparin followed by rPF4 (Fig. 2). In these Figs., heparin was injected at t=0 and either protamine sulfate or rPF4 injected two minutes thereafter (t=2 minutes). The dosages used were: heparin 1 unit/g animal wt.; protamine sulfate 1 ug/g animal wt.; and PF4 7.5 ug/g animal wt.

In Fig. 1, the effect of protamine is shown to produce a decrease in the APTT to approximately normal levels after about 10 minutes. There was also observed a significant decrease of white blood cell count and platelet count after injection with protamine sulfate. Ten minutes after injection of heparin and eight minutes after injection of protamine sulfate, platelet count had fallen from over 800 x 10³/µl (measured at t=0) to less than 500 x 10³/µl (measured at t=10 minutes). Similarly, white blood count fell from over 5 x 10³/µl (t=0) to less than about 3 x 10³/µl (t=10 minutes). These rats also had respiratory problems.

Administration of rPF4 also effectively neutralized heparin, as shown by restoration of normal APTT levels at t=10 minutes (ten minutes after injection of heparin and eight minutes after injection of rPF4; Figure 2). Neutralization of heparin by rPF4 did not lead to the extensive depletion of platelet and white blood cells as caused by protamine sulfate. Platelet count remained relatively stable falling only from the initial value of 700 x 10³/µl (t=0) to about 500 x 10³/µl (t=10 minutes). White blood cell count remained relatively stable at about 4 x 10³/µl throughout the tests.

Experiments were also conducted to investigate hematocrit levels under conditions as described with respect to Figs. 1 and 2. No change in hematocrit levels were observed when either PF4 or protamine were used for heparin neutralization.

In addition, three experiments were performed in which heparinized rats were injected with low doses of rPF4 (150 µg/100g). In these rats the anticoagulant effect of heparin was partially neutralized and no effect on platelets and white blood cells was observed.

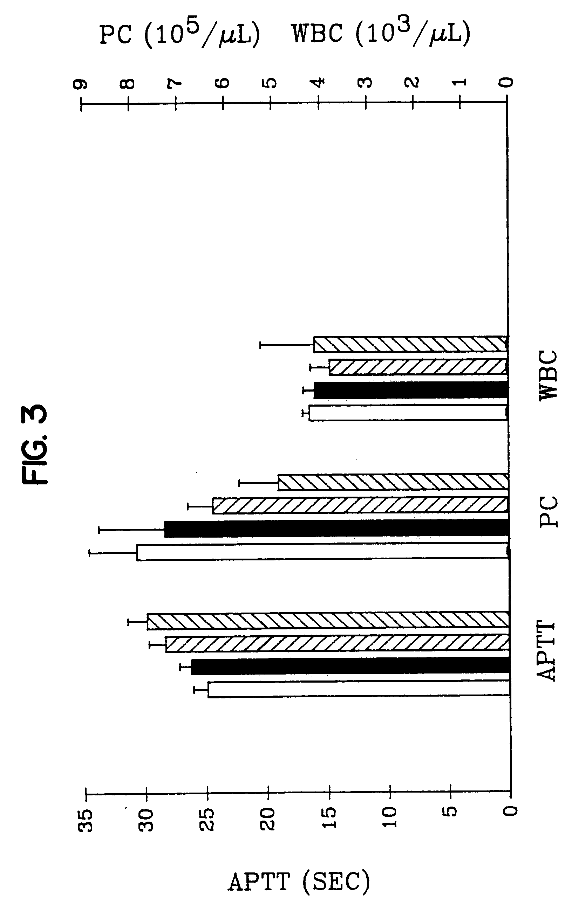

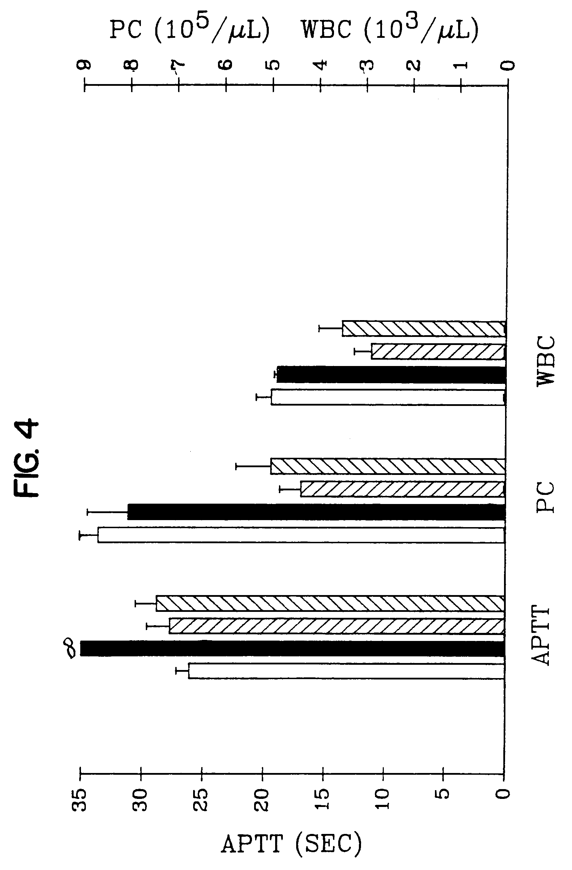

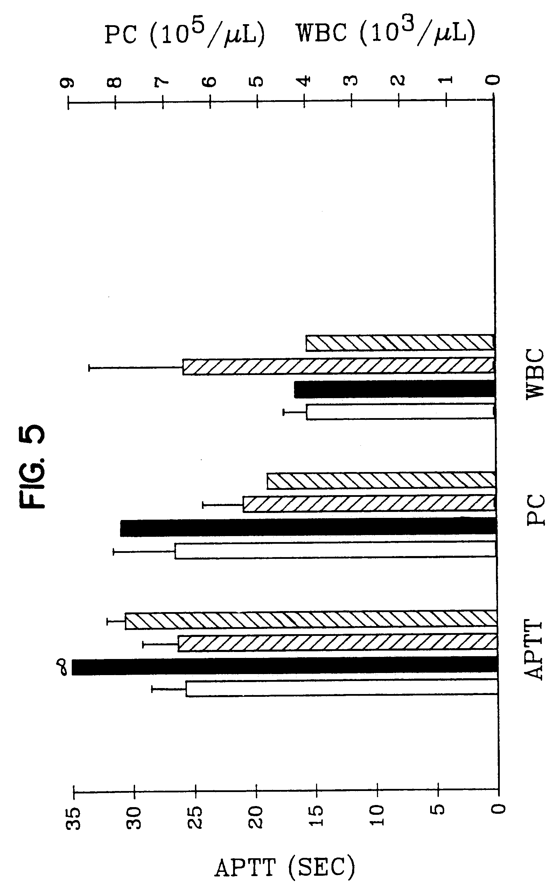

Referring now to Figs. 3 to 5, statistical bar graph data are shown comparing APTT, platelet count, (PC) and white blood cell (WBC) count in rats injected with a control solution, Ringers solution (Fig. 3), injected with heparin followed by protamine sulfate (Fig. 4) and injected with heparin followed by rPF4 (Fig. 5). The data included control rats, five rats injected with heparin followed by protamine sulfate and two rats injected with heparin followed by rPF4.

Fig. 3 shows APTT, PC and WBC in rats after injection of mammalian Ringer's solution. Samples A were taken before injection; samples B, 2 min after injection; samples C, 10 min after injection; and samples D, 18 min after injection. Data represents mean values and S.E.M. from 5 experiments.

Fig. 4 shows APTT, PC and WBC in rats after injection of heparin (100 units/100g) followed by protamine sulfate (100µg/100g). Samples A were taken before injection; samples B, 2 min after heparin injection; samples C, 10 min after heparin and 8 min after protamine sulfate injection; and samples D, 18 min after heparin injection. Data represent mean values and S.E.M. from experiments except that white blood cells and platelets were counted only in 3 samples taken at 2 and at 18 min after heparin injection. The data in Fig. 4 indicate a drop in platelet and white blood cell counts in samples after protamine sulfate injection to heparinized rats. The decrease was statistically significant (p<0.01) as compared to samples obtained before heparin injection.

Fig. 5 shows APTT, PC, and WBC in rats after injection of heparin followed by rPF4 (7.5 µg/g). Data represent mean values and S.E.M. from 2 experiments for samples taken at times A-D as indicated in the discussion above with respect to Figure 4. The injection of rPF4 did not produce a statistical decrease in platelet and white blood count.

As evident in the comparision of Figs. 3, 4 and 5, platelet count remains relatively constant when rPF4 is administered to neutralize heparin (Figure 5) compared to when protamine is administered to neutralize heparin (Figure 4). Statistically, platelet count decreased significantly in the presence of protamine (48±12.2% of initial) but remains normal following rPF4 (89±4.9%). WBC count also decreased following protamine sulfate treatment. These results indicate that PF4 is superior to protamine in heparin neutralization in a number of respects.

Purified rPF4 is prepared by a rigourously controlled procedure and is substantially free of contaminants.

PF4 can be administered intravenously following heparin treatment, for example, using a slow drip i.v. infusion over the course of 10 minutes. PF4 may be employed following heparin treatment in any surgical procedure in which heparin is employed or therapeutically when heparin is used to avoid clotting. Accordingly a human patient may be treated during a medical procedure by first administering to that patient a therapeutically effective amount of heparin to inhibit coagulation, and then subsequently administering to the same patient a therapeutically effective amount of purified PF4 or a heparin-binding fragment thereof to neutralize heparin. A typical effective dose of rPF4 is approximately 6.35 mg per kg of body weight, based on an effective protamine dose of approximately 3.6 mg per kg of body weight. However, the actual dosage may vary with the dosage of heparin.

## Claims

1. Use of purified PF4 or a heparin binding fragment thereof for the manufacture of a medicament for treatment of an undesirably high level of circulating heparin in a mammal caused by administration of heparin in connection with a medical procedure.

2. Use as defined in Claim 1 of PF4 or a heparin binding fragment thereof, wherein said PF4 or fragment thereof is rPF4 or a fragment thereof.

3. Use as defined in Claim 1 of PF4 or a heparin binding fragment thereof, wherein said PF4 or fragment thereof is synthetic PF4 or a fragment thereof.

4. Use as defined in Claim 1 wherein said heparin binding fragment of PF4 contains amino acids 61 to 66.

5. Use as defined in Claim 4 wherein said fragment is C-41.

6. Use of purified PF4 or a heparin binding fragment thereof for the manufacture of a medicament for restoring a normal coagulation status in a patient after administration of heparin, without substantial depletion of platelets or leukocytes.

7. Use of purified PF4 or a heparin binding fragment thereof for the manufacture of a medicament for neutralization of heparin administered to a patient to inhibit coagulation during a medical treatment.

8. Use as defined in Claim 7 of PF4 or a heparin binding fragment thereof, wherein said inhibition of coagulation is during heart surgery, cardiopulmonary bypass, organ transplantation, kidney dialysis, catheterization or for the prevention of blood clot formation.

9. Use as defined in Claim 7 of PF4 or a heparin binding fragment thereof, wherein said inhibition of coagulation is during catheterization, said catheterization being cardiac catheterization, diagnostic or for angioplasty.

10. The combination of heparin and purified PF4 or a heparin binding fragment thereof for the spaced sequential use in the treatment of a patient during a medical procedure, said heparin inhibiting coagulation and said purified PF4 or heparin binding fragment thereof neutralizing said heparin.

11. A combination according to Claim 10 for sequential use in the treatment of a patient during a medical procedure wherein said medical procedure is one of heart surgery, a cardiopulmonary bypass, an organ transplantation, a kidney dialysis, a catheterization and one for the prevention of blood clot formation.

12. A combination according to Claim 11 for sequential use in the treatment of a human patient during a catheterization wherein said catheterization is a cardiac catheterization, diagnostic or for angioplasty.

## Patentansprüche

1. Verwendung von gereinigtem PF4 oder einem Heparin-bindenden Fragment davon zur Herstellung eines Medikaments zur Behandlung eines unerwünscht hohen Spiegels zirkulierenden Heparins in einem Säuger, verursacht durch Verabreichung von Heparin in Verbindung mit einem medizinischen Verfahren.

2. Verwendung von PF4 oder einem Heparin-bindenden Fragment davon nach Anspruch 1, dadurch **gekennzeichnet,** daß PF4 oder ein Fragment davon rPF4 oder ein Fragment davon ist.

3. Verwendung von PF4 oder einem Heparin-bindenden Fragment davon nach Anspruch 1, dadurch **gekennzeichnet,** daß PF4 oder ein Fragment davon synthetisches PF4 oder ein Fragment davon ist.

4. Verwendung nach Anspruch 1, dadurch **gekennzeichnet,** daß das Heparin-bindende Fragment von PF4 die Aminosäuren 61 bis 66 enthält.

5. Verwendung nach Anspruch 4, dadurch **gekennzeichnet,** daß das Fragment C-41 ist.

6. Verwendung von gereinigtem PF4 oder einem Heparin-bindenden Fragment davon zur Herstellung eines Medikaments zur Wiederherstellung eines normalen Gerinnungsstatus in einem Patienten nach Verabreichung von Heparin, ohne wesentliche Depletion von Blutplättchen oder Leukozyten.

7. Verwendung von gereinigtem PF4 oder einem Heparin-bindenden Fragment davon zur Herstellung eines Medikaments zur Neutralisation von Heparin, das einem Patienten verabreicht wurde, um die Gerinnung während einer medizinischen Behandlung zu hemmen.

8. Verwendung von PF4 oder einem Heparin-bindenden Fragment davon nach Anspruch 7, dadurch **gekennzeichnet,** daß die Inhibierung der Gerinnung während Herzoperation, kardiopulmonärem Bypass, Organtransplantation, Nierendialyse, Katheterisierung oder zur Vorbeugung einer Blutgerinselbildung stattfindet.

9. Verwendung von PF4 oder einem Heparin-bindenden Fragment davon nach Anspruch 7, dadurch **gekennzeichnet,** daß die Inhibierung der Gerinnung während einer Katheterisierung stattfindet, wobei die Katheterisierung eine Herzkatheterisierung, zur Diagnose oder für eine Gefäßplastik ist.

10. Kombination von Heparin und gereinigtem PF4 oder einem Heparin-bindenden Fragment davon für zeitlich auseinanderliegende Verwendung in Folge bei der Behandlung eines Patienten während eines medizinischen Verfahrens, wobei das Heparin die Gerinnung hemmt und das gereinigte PF4 oder das Heparin-bindende Fragment davon das Heparin neutralisiert.

11. Kombination nach Anspruch 10 für die Verwendung in Folge in der Behandlung eines Patienten während eines medizinischen Verfahrens, wobei das medizinische Verfahren ausgewählt ist aus Herzoperation, kardiopulmonärem Bypass, Organtransplantation, Nierendialyse, Katheterisierung und Vorbeugung einer Blutgerinselbildung.

12. Kombination nach Anspruch 11 zur Verwendung in Folge in der Behandlung eines menschlichen Patienten während einer Katheterisierung, wobei die Katheterisierung eine Herzkatheterisierung, zur Diagnose oder für eine Gefäßplastik ist.

## Revendications

1. Utilisation du facteur plaquettaire 4 (FP 4) purifié ou d'un de ses fragments se liant à l'héparine pour la production d'un médicament destiné au traitement d'un taux trop élevé d'héparine circulante chez un mammifère dû à l'administration d'héparine dans le cadre d'un acte médical.

2. Utilisation de FP 4 ou d'un de ses fragments se liant à l'héparine selon la revendication 1, dans laquelle ledit FP 4 ou son fragment est un rFP 4 (FP 4 recombiné) ou un de ses fragments.

3. Utilisation de FP 4 ou d'un de ses fragments se liant à l'héparine selon la revendication 1, dans laquelle ledit FP 4 ou son fragment est un FP 4 synthétique ou un de ses fragments.

4. Utilisation selon la revendication 1, dans laquelle ledit fragment de FP 4 se liant à l'héparine contient les aminoacides 61 à 66.

5. Utilisation selon la revendication 4, dans laquelle ledit fragment est le C-41.

6. Utilisation de FP 4 purifié ou d'un de ses fragments se liant à l'héparine pour la production d'un médicament destiné au rétablissement d'un état de coagulation normal chez un patient après l'administration d'héparine, sans déplétion importante de plaquettes ni de leucocytes.

7. Utilisation de FP 4 purifié ou d'un de ses fragments se liant à l'héparine pour la production d'un médicament destiné à la neutralisation de l'héparine administrée à un patient pour inhiber la coagulation pendant un traitement médical.

8. Utilisation de FP 4 purifié ou d'un de ses fragments se liant à l'héparine selon la revendication 7, dans laquelle ladite inhibition de la coagulation a lieu au cours d'une intervention de chirurgie cardiaque, d'un pontage cardio-pulmonaire, d'une greffe d'organes, d'une dialyse rénale, d'un cathétérisme, ou pour la prévention de la formation d'un caillot sanguin.

9. Utilisation de FP 4 purifié ou d'un de ses fragments se liant à l'héparine selon la revendication 7, dans laquelle ladite inhibition de la coagulation a lieu au cours d'un cathétérisme, ledit cathétérisme étant un cathétérisme cardiaque, de diagnostic ou destiné à une angioplastie.

10. Combinaison d'héparine et de FP 4 ou d'un de ses fragments se liant à l'héparine, à utiliser successivement de manière espacée dans le traitement d'un patient au cours d'un acte médical, ladite héparine inhibant la coagulation et ledit FP 4 ou son fragment se liant à l'héparine neutralisant ladite héparine.

11. Combinaison selon la revendication 10, à utiliser successivement dans le traitement d'un patient au cours d'un acte médical, ledit acte médical étant un opération de chirurgie cardiaque, un pontage cardio-pulmonaire, une greffe d'organes, une dialyse rénale, un cathétérisme et un acte destiné à prévenir la formation d'un caillot sanguin.

12. Combinaison selon la revendication 11, à utiliser successivement dans le traitement d'un patient humain au cours d'un cathétérisme, ledit cathétérisme étant un cathétérisme cardiaque, de diagnostic ou destiné à une angioplastie.
